(19) Europäisches Patentamt / European Patent Office / Office européen des brevets

(11) **EP 3 541 511 B1**

(12) **EUROPÄISCHE PATENTSCHRIFT**

(45) Veröffentlichungstag und Bekanntmachung des Hinweises auf die Patenterteilung:
**27.08.2025 Patentblatt 2025/35**

(21) Anmeldenummer: **17801389.2**

(22) Anmeldetag: **09.11.2017**

(51) Internationale Patentklassifikation (IPC):
**B01J 37/00** (2006.01)   **B01J 37/03** (2006.01)
**B01J 37/18** (2006.01)   **B01J 37/14** (2006.01)
**B01J 35/36** (2024.01)   **B01J 35/32** (2024.01)
**B01J 23/80** (2006.01)   **B01J 23/00** (2006.01)
**C07C 29/154** (2006.01)

(52) Gemeinsame Patentklassifikation (CPC):
(C-Sets verfügbar)
**B01J 23/80; B01J 23/007; B01J 35/32;**
**B01J 35/36; B01J 35/50; B01J 37/0063;**
**B01J 37/14; B01J 37/18; C07C 29/154;**
B01J 37/031; B01J 2235/00; B01J 2235/10  (Forts.)

(86) Internationale Anmeldenummer:
**PCT/EP2017/078703**

(87) Internationale Veröffentlichungsnummer:
**WO 2018/091342 (24.05.2018 Gazette 2018/21)**

(54) **VERFAHREN ZUR HERSTELLUNG MECHANISCH STABILER KATALYSATOREN ZUR HYDRIERUNG VON CARBONYLVERBINDUNGEN**

METHOD FOR PRODUCING MECHANICALLY STABLE CATALYSTS FOR HYDROGENATING CARBONYL COMPOUNDS

PROCÉDÉ DE FABRICATION DE CATALYSEURS MÉCANIQUEMENT STABLES POUR L'HYDROGÉNATION DE COMPOSÉS CARBONYLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.11.2016 EP 16198921**

(43) Veröffentlichungstag der Anmeldung:
**25.09.2019 Patentblatt 2019/39**

(73) Patentinhaber: **BASF SE**
**67056 Ludwigshafen am Rhein (DE)**

(72) Erfinder:
• **HEIDEMANN, Thomas**
**67056 Ludwigshafen (DE)**
• **WUCHER, Barbara**
**67056 Ludwigshafen (DE)**
• **KOELKER, Thomas**
**40589 Düsseldorf-Holthausen (DE)**
• **PEPERS, Michel**
**40589 Düsseldorf-Holthausen (DE)**
• **BRINKMANN, Ulf**
**40589 Düsseldorf-Holthausen (DE)**
• **TOPPHOFF, Magnus**
**40589 Düsseldorf-Holthausen (DE)**
• **JOSTEN, Horst**
**40593 Düsseldorf (DE)**

(74) Vertreter: **BASF IP Association**
**BASF SE**
**GBI - Z078**
**67056 Ludwigshafen (DE)**

(56) Entgegenhaltungen:
**DE-A1- 10 357 715     DE-A1- 102012 019 123**

**(Forts. nächste Seite)**

(52) Gemeinsame Patentklassifikation (CPC): (Forts.)

C-Sets
**C07C 29/154, C07C 31/125;**
**C07C 29/154, C07C 33/02**

## Beschreibung

### Gebiet der Erfindung

Die vorliegende Erfindung betrifft ein Verfahren zur Herstellung eines metallcarbonat-haltigen Katalysatorformkörpers für die Hydrierung einer eine oder mehrere Carbonylgruppe(n) aufweisenden organischen Verbindung.

### Stand der Technik

Die katalytische Hydrierung von Carbonylverbindungen, wie Aldehyden, Ketonen, Carbonsäuren und Carbonsäureestern, spielt in der chemischen Industrie eine wichtige Rolle, insbesondere in den Prozessen zur Herstellung von Grundchemikalien. Bei der katalytischen Hydrierung werden verschiedenste homogene und heterogene Hydrierkatalysatoren eingesetzt. Großtechnisch von Bedeutung sind insbesondere heterogene Hydrierkatalysatoren, die üblicherweise in verschiedener dreidimensionaler Ausgestaltung, z.B. als Pulver, Granulat oder definierte Formkörper, in Wirbelschicht- oder Festbettverfahren eingesetzt werden. Am gängigsten sind hierbei Festbettverfahren, bei denen in der Regel definierte Katalysatorformkörper zum Einsatz kommen.

In den Festbettverfahren werden die Katalysatorformkörper üblicherweise hohen mechanischen Belastungen ausgesetzt. Eine geringe mechanische Stabilität der Katalysatorformkörper führt in der Regel zu einem vorzeitigen Katalysatorzerfall und damit zu einer reduzierten Einsatzdauer bzw. häufigen Ausfällen der mit den Katalysatorformkörpern bestückten Hydrierreaktoren. Um einen übermäßigen Katalysatorzerfall zu verhindern, muss die mechanische Belastung möglichst gering gehalten werden, was in der Regel zu einer geringeren Reaktorauslastung führt. Neben der katalytischen Aktivität hat somit auch die mechanische Stabilität der eingesetzten Katalysatorformkörper einen erheblichen Einfluss auf die Wirtschaftlichkeit katalytischer Hydrierverfahren.

Zur Erhöhung der katalytischen Aktivität und/oder der mechanischen Stabilität von Katalysatorformkörpern werden im Stand der Technik verschiedene Ansätze verfolgt.

Die DE 102014004413 A1 beschreibt beispielsweise die Herstellung tablettierter Katalysatorformkörper zur Hydrierung von Carbonylverbindungen ausgehend von metallcarbonathaltigen Gemischen, die gegenüber den im Stand der Technik bekannten Hydrierkatalysatoren eine höhere Metalloberfläche und damit eine höhere Aktivität aufweisen. Speziell wird ein Verfahren zur Herstellung von Cu-Zn-Katalysatorformkörpern beschrieben, bei dem ein mittels Fällung erhältliches Gemisch aus Cu- und Zink-Carbonat einer thermischen Behandlung bei einer Temperatur im Bereich von 150°C bis 350°C unterzogen wird, wobei ein Cu-Zn-Carbonatgemisch mit einem Molverhältnis von Cu zu Zn von etwa 1 : 2 und mit einem Carbonatgehalt von 2,7 bis 14,0 Gew.-% erhalten wird, welches anschließend tablettiert wird.

Die EP 2096098 A1 beschreibt ein Verfahren zur Herstellung tablettierter Katalysatorformkörper mit sowohl hoher mechanischer Stabilität als auch hoher Hydrieraktivität, bei dem man Verbindungen von Kupfer und Zink, insbesondere Kupfer- und Zinkcarbonat, gemeinsam auf ein Trägermaterial aus Aluminiumoxidpulver ausfällt, das so erhaltene Katalysatormaterial kalziniert, mit 5 Gew.-% Graphit vermischt, tablettiert und die fertigen Katalysatortabletten nachkalziniert.

Die DE 102012019123 A1 beschreibt ein Verfahren zur Herstellung tablettierter Cu-Al-Katalysatorformkörper mit einer hohen Seitendruckfestigkeit und einer hohen Hydrieraktivität, bei dem zunächst ein trockenes carbonathaltiges Gemisch, das Cu, Al und Mn enthält, bereitgestellt wird, wobei ein Teil dieses carbonathaltigen Gemisches kaliziniert wird. Das unkalzinierte, trockene carbonathaltige Gemisch wird nach Zugabe von Graphit entweder direkt tablettiert oder anteilig mit dem kalzinierten carbonathaltigen Gemisch gemischt und dann nach Zugabe von Graphit tablettiert. Optional werden die so erhaltenen Cu-Al-Katalysatorformkörper nachkalziniert.

Durch die Kalzinierung können sehr harte und damit mechanisch stabile Katalysatorformkörpern erhalten werden. Jedoch führen Temperaturbehandlungen in der Regel immer zu einer mehr oder weniger starken Sinterung, durch die die Größe der aktiven Katalysatoroberfläche und damit die Hydrieraktivität der Katalysatoren verringert wird. Um Hydrierkatalysatoren mit einer möglichst hohen Aktivität zu erhalten, müssen die durch die Temperaturbehandlung hervorgerufenen Sinterungseffekte in der Regel möglichst gering gehalten werden.

Die DE 10357715 A1 beschreibt ein Verfahren zur Herstellung eines Hydrierkatalysators, bei dem ein Gemisch aus Kupfer- und Zinkcarbonat in Anwesenheit eines Trägers, bestehend aus Aluminiumoxid und Zinkoxid, ausgefällt wird. Die Suspension wird anschließend abfiltriert, getrocknet und kalziniert. Das kalzinierte Katalysatorpulver wird mit 1,5 Gew.-% Graphit und 5 Gew.-% Kupferpulver gemischt, tablettiert und nachkalziniert. Die so erhaltenen Katalysatorformkörper weisen eine Zusammensetzung von 66% CuO, 24% ZnO, 5% $Al_2O_3$ und 5% Cu auf.

Die WO 01/17934 A1 beschreibt eine Verfahren zur Herstellung tablettierter Katalysatorformkörper mit sowohl hoher mechanischer Stabilität als auch hoher Hydrieraktivität, bei dem zu einer Mischung aus verschiedenen Metalloxiden pulverförmiges metallisches Kupfer und/oder pulverförmiger Zement als Bindemittel zugegeben wird und das so erhaltene Gemisch anschließend zu einem Formkörper verformt wird.

Durch den Zusatz von Bindemitteln wie Metallpulvern, beispielsweise metallisches Kupferpulver, und/oder Zement zum Katalysatormaterial kann die mechanische Stabilität der Katalysatorformkörper erhöht werden. Dies führt jedoch zu einer Reduktion der katalytischen Aktivität der daraus hergestellten Katalysatorformkörper, da durch die Zugabe des Bindemittels die Menge an aktiver Katalysatormasse reduziert wird.

**Beschreibung der Erfindung**

Der vorliegenden Erfindung liegt die Aufgabe zugrunde, ein Verfahren zur Herstellung von Katalysatorformkörpern zur Hydrierung von Carbonylverbindungen ausgehend von metallcarbonathaltigen Gemischen bereitzustellen, die einerseits mechanisch stabil sind, d.h. eine hohe Seitendruckfestigkeit aufweisen, und andererseits eine, gegenüber Katalysatorformkörpern, die auf demselben Katalysatormaterial basieren aber weniger stabil sind, zumindest ebenbürtige Hydrieraktivität aufweisen.

Es wurde nun überraschenderweise gefunden, dass die Zugabe von metallischem Kupfer zu einem Metallcarbonatgemisch, enthaltend zwei oder mehr als zwei Metallcarbonate von zwei oder mehr als zwei verschiedenen Metallen, nach der Tablettierung und Aktivierung zu Katalysatorformkörpern führt, die gegenüber Katalysatorformkörpern, die aus demselben Metallcarbonatgemisch aber ohne die Zugabe von metallischem Kupfer hergestellt wurden, nicht nur eine wesentlich erhöhte Stabilität sondern sogar eine erhöhte Hydrieraktivität aufweisen.

Dementsprechend betrifft ein erster Gegenstand der vorliegenden Erfindung ein Verfahren zur Herstellung eines Katalysatorformkörpers, bei dem man

a) eine metallcarbonathaltige Masse bereitstellt, die, bezogen auf das Gesamtgewicht der metallcarbonathaltigen Masse,

- 70 Gew.-% oder mehr eines Metallcarbonatgemisches, enthaltend zwei oder mehr als zwei Metallcarbonate von zwei oder mehr als zwei verschiedenen Metallen (M),
- 10 bis 25 Gew.-% metallisches Kupfer und
- 0,5 bis 5 Gew.-% Tablettierhilfsmittel,

enthält,

b) aus der in Schritt a) bereitgestellten metallcarbonathaltigen Masse einen Formkörper formt und

c) den in Schritt b) erhaltenen Formkörper bei einer Temperatur im Bereich von 150 bis 250 °C in Gegenwart von Wasserstoff aktiviert.

Des Weiteren wurde gefunden, dass die Hydrieraktivität der Katalysatorformkörper weiter erhöht werden kann, wenn der nach der Tablettierung erhaltene Katalysatorformkörper vor der Aktivierung in Gegenwart von Wasserstoff einer thermischen Behandlung in Abwesenheit von Wasserstoff bei einer Temperatur im Bereich von 150 bis 350 °C unterworfen wird.

Daher betrifft ein zweiter Gegenstand der vorliegenden Erfindung ein Verfahren zur Herstellung eines Katalysatorformkörpers, wie oben definiert, wobei der in Schritt b) erhaltenen Formkörper zunächst in einem Schritt c1) einer ersten thermischen Behandlung bei einer Temperatur im Bereich von 150 bis 350 °C in Abwesenheit von Wasserstoff unterworfen wird und der so thermisch behandelte Formkörper anschließend in einem Schritt c2) bei einer Temperatur im Bereich von 150 bis 250 °C in Gegenwart von Wasserstoff aktiviert wird.

Offenbart ist ein Verfahren zur Hydrierung einer eine oder mehr als eine Carbonylgruppe(n) aufweisenden organischen Verbindung, bei dem die organische Verbindung in Gegenwart von Wasserstoff mit einem Katalysatorformkörper, erhältlich durch eines der zuvor und nachfolgend definierten Verfahren, in Kontakt gebracht wird.

**Zu Schritt a) des Verfahrens**

In Schritt a) des erfindungsgemäßen Verfahrens zur Herstellung eines Katalysatorformkörpers, wird eine metallcarbonathaltige Masse bereitgestellt, die, bezogen auf das Gesamtgewicht der metallcarbonathaltigen Masse,

- 70 Gew.-% oder mehr eines Metallcarbonatgemisches, enthaltend zwei oder mehr als zwei Metallcarbonate von zwei oder mehr als zwei verschiedenen Metallen (M),
- 10 bis 25 Gew.-% metallisches Kupfer und
- 0,5 bis 5 Gew.-% Tablettierhilfsmittel,

enthält.

Die in Schritt a) bereitgestellte metallcarbonathaltige Masse enthält als Hauptkomponente 70 Gew.-% oder mehr, bevorzugt 76 Gew.-% oder mehr, insbesondere 82 bis 89 Gew.-%, eines Metallcarbonatgemisches.

Erfindungsgemäß enthält das in Schritt a) eingesetzte Metallcarbonatgemisch zwei oder mehr als 2 Metallcarbonate von zwei oder mehr als zwei verschiedenen Metallen (M).

Bevorzugt enthält das in Schritt a) eingesetzte Metallcarbonatgemisch 2 bis 10 Metallcarbonate von 2 bis 10 verschiedenen Metallen (M).

Besonders bevorzugt enthält das in Schritt a) eingesetzte Metallcarbonatgemisch 2 bis 5 Metallcarbonate von 2 bis 5 verschiedenen Metallen (M).

Insbesondere enthält das in Schritt a) eingesetzte Metallcarbonatgemisch 2 bis 5 Metallcarbonate von 2 oder 3 verschiedenen Metallen (M).

Die Metallcarbonate, die zur Herstellung der metallcarbonathaltigen Masse eingesetzt werden, können als "reine" Metallcarbonate der allgemeinen Formel $M_{2n/m}^{m+}(CO_3^{2-})_n$ , wobei n und m für eine ganze Zahl von 1 bis 4 stehen, und/oder in Form von Metallhydroxycarbonaten, sogenannten basischen Metallcarbonaten, der allgemeinen Formel $x[M_{2n/m}^{m+}(CO_3^{2-})_n] \bullet y[M_{l/m}^{m+}(OH^-)_l]$ , wobei l, m, n, x und y für eine ganze Zahl von 1 bis 4 stehen, vorliegen. Zudem können sowohl die "reinen" Metallcarbonate als auch die Metallhydroxycarbonate unterschiedliche Mengen Kristallwasser enthalten.

Dementsprechend umfasst der Ausdruck "Metallcarbonate" im Rahmen der vorliegenden Erfindung Metallcarbonate und/oder Metallhydroxycarbonate sowie deren Hydrate.

Die Metallcarbonate, die zur Herstellung der metallcarbonathaltigen Masse eingesetzt werden, können als "reine" Metallcarbonate der allgemeinen Formel $M^{2+}(CO_3^{2-})$ und/oder als Metallhydroxycarbonate der allgemeinen Formel $x[M^{2+}(CO_3^{2-})] \bullet y[M^{2+}(OH^-)_2]$ , wobei x und y für eine ganze Zahl von 1 bis 3 stehen, vorliegen. Zudem können sowohl die "reinen" Metallcarbonate als auch die Metallhydroxycarbonate unterschiedliche Mengen Kristallwasser enthalten.

Als Metall (M) können in der Regel alle dem Fachmann bekannten Metalle, die sich zur Hydrierung von Carbonylgruppen eignen, eingesetzt werden. Bevorzugt ist das Metall (M) ausgewählt unter Übergangsmetalle der 8., 9., 10., 11. und 12. Gruppe des Periodensystems nach IUPAC. Besonders bevorzugt ist das Metall (M) ausgewählt unter Co, Ni, Cu, Au, Zn, Cd, Ru und Fe, insbesondere unter Cu und Zn.

In einer bevorzugten Ausführungsform des erfindungsgemäßen Verfahrens zur Herstellung eines Katalysatorformkörpers, enthält das in der metallcarbonathaltigen Masse enthaltene Metallcarbonatgemisch, bezogen auf das Gesamtgewicht des Metallcarbonatgemisches,

- 40 bis 65 Gew.-% Kupfercarbonat
- 35 bis 60 Gew.-% Zinkcarbonat und
- 0 bis 20 Gew.-% eines von Kupfercarbonat und Zinkcarbonat verschiedenen Metallcarbonats.

Bei dem im Metallcarbonatgemisch enthaltenen Kupfercarbonat handelt es sich üblicherweise um basisches Kupfercarbonat, d.h. um Kupferhydroxycarbonate, wie beispielsweise $[Cu(CO_3)] \bullet [Cu(OH)_2]$ und/oder $2[Cu(CO_3)] \bullet [Cu(OH)_2]$.

Bei dem im Metallcarbonatgemisch enthaltenen Zinkcarbonat handelt es sich üblicherweise um Zinkcarbonate, wie beispielsweise $Zn(CO_3)$ und/oder $Zn_2(CO_3)$, und/oder um basisches Zinkcarbonat, d.h. um Zinkhydroxycarbonate, wie beispielsweise $2[Zn(CO_3)] \bullet 3[Zn(OH)_2]$.

Bevorzugt ist das von Kupfercarbonat und Zinkcarbonat verschiedene Metallcarbonat ausgewählt unter Cobalt-, Nickel-, Gold-, Cadmium-, Ruthenium- und Eisencarbonat.

Das in der vorliegenden Erfindung eingesetzte Metallcarbonatgemisch kann weitere von Metallcarbonaten verschiedene Metallverbindungen, insbesondere von Metallcarbonaten verschiedene Metallsalze, als Verunreinigungen enthalten oder/und dem Metallcarbonatgemisch kann/können eine oder mehrere von Metallcarbonaten verschiedene Metallverbindung(en), insbesondere (ein) von Metallcarbonaten verschiedene(s) Metallsalz(e), zugesetzt sein. Falls dem Metallcarbonatgemisch eine oder mehrere von Metallcarbonaten verschiedene Metallverbindung(en), insbesondere ein oder mehrere von Metallcarbonaten verschiedene(s) Metallsalz(e), zugesetzt wird, so beträgt deren Anteil am Gesamtgewicht des Metallcarbonatgemisches maximal 25 Gew.-%, bevorzugt maximal 15 Gew.-%, besonders bevorzugt maximal 10 Gew.-% und insbesondere maximal 5 Gew.-%.

Bei den von Metallcarbonaten verschiedenen Metallverbindungen, die in dem Metallcarbonatgemisch als Verunreinigungen enthalten sein können, handelt es sich üblicherweise um von Metallcarbonaten verschiedene Metallsalze, die als Ausgangsstoffe zur Herstellung der Metallcarbonate eingesetzt werden. Hierbei handelt es sich häufig um die Nitrate, Halogenide, wie beispielsweise die Chloride, Bromide und/oder Iodide, Oxide, Hydroxide, Acetate, Phosphate und/oder Sulfate von Co, Ni, Cu, Au, Zn, Cd, Ru, Fe, Ca, Ba, Ce, Ti, Zr, Cr, Mo, Mn, Sn, Al, Si, sowie um Gemische davon.

In diesem Zusammenhang bedeutet der Begriff "Verunreinigung(en)", dass die Menge der oben aufgeführten von Metallcarbonaten verschiedenen Metallverbindungen in dem Metallcarbonatgemisch maximal 1 Gew.-%, bevorzugt maximal 0,5 Gew.-%, insbesondere maximal 0,1 Gew.-%, bezogen auf das Gesamtgewicht des Metallcarbonatgemisches, beträgt.

Falls dem Metallcarbonatgemisch eine oder mehrere von Metallcarbonaten verschiedene Metallverbindung(en) zugesetzt wird, ist/sind die zugesetzte(n) von Metallcarbonaten verschiedene Metallverbindung(en) bevorzugt ausgewählt unter Nitraten, Halogeniden, wie beispielsweise den Chloriden, Bromiden und/oder Iodiden, Oxiden, Hydroxiden, Acetaten, Phosphaten und Sulfaten von Ca, Ba, Ce, Ti, Zr, Cr, Mo, Mn, Sn, Al und Si, insbesondere unter Nitraten, Halogeniden, wie beispielsweise den Chloriden, Bromiden und/oder Iodiden, Oxiden, und Sulfaten von Ce, Zr, Mn und Al.

Speziell wird zu dem in der vorliegenden Erfindung eingesetzten Metallcarbonatgemisch keine weitere von Metallcarbonaten verschiedene Metallverbindung zugesetzt. Selbstverständlich können in dem in der vorliegenden Erfindung eingesetzte Metallcarbonatgemisch dennoch geringe Mengen an von Metallcarbonaten verschiedenen Metallverbindungen, insbesondere von Metallcarbonaten verschiedenen Metallsalzen, wie oben definiert, als Verunreinigungen enthalten.

In der Regel liegt die Menge an Metallcarbonaten, wie oben definiert, in dem Metallcarbonatgemisch bei 75 Gew.-% oder höher, bevorzugt bei 85 Gew.-% oder höher, bezogen auf das Gesamtgewicht des Metallcarbonatgemisches.

Insbesondere liegt die Menge an Metallcarbonaten, wie oben definiert, in dem Metallcarbonatgemisch bei 90 Gew.-% oder höher, beispielsweise bei 95 Gew.-% oder 97 Gew.-%, bezogen auf das Gesamtgewicht des Metallcarbonatgemisches.

In einer besonders bevorzugten Ausführungsform des erfindungsgemäßen Verfahrens zur Herstellung eines Katalysatorformkörpers, besteht das in der metallcarbonathaltigen Masse enthaltene Metallcarbonatgemisch aus

- 40 bis 65 Gew.-% Kupfercarbonat und
- 35 bis 60 Gew.-% Zinkcarbonat.

Auch in dieser besonders bevorzugten Ausführungsform kann das Metallcarbonatgemisch dennoch geringe Mengen an von Kupfer- und Zinkcarbonat verschiedenen Kupfer- und Zinkverbindungen, insbesondere von Kupfer- und Zinkcarbonat verschiedenen Kupfer- und Zinksalzen, als Verunreinigungen enthalten.

Falls in dem Metallcarbonatgemisch Kupfer- und Zinkcarbonat enthalten ist oder das Metallcarbonatgemisch aus Kupfer- und Zinkcarbonat besteht, liegt das Molverhältnis von Kupfercarbonat und Zinkcarbonat im Metallcarbonatgemisch üblicherweise im Bereich von 2 : 1 bis 1 : 1,5, bevorzugt im Bereich von 1,7 : 1 bis 1 : 1,2, insbesondere im Bereich von 1,5 : 1 bis 1 : 1.

In einer weiteren bevorzugten Ausführungsform des erfindungsgemäßen Verfahrens zur Herstellung eines Katalysatorformkörpers, enthält das in der metallcarbonathaltigen Masse enthaltene Metallcarbonatgemisch, bezogen auf das Gesamtgewicht des Metallcarbonatgemisches,

- 22 bis 37 Gew.-% Kupfer und
- 18 bis 33 Gew.-% Zink.

Besonders bevorzugt enthält das in der metallcarbonathaltigen Masse enthaltene Metallcarbonatgemisch, bezogen auf das Gesamtgewicht des Metallcarbonatgemisches,

- 25 bis 34 Gew.-% Kupfer und
- 20 bis 30 Gew.-% Zink.

Das in dem Metallcarbonatgemisch enthaltene Kupfer und Zink liegt überwiegend als Kupfercarbonat und Zinkcarbonat, wie oben definiert, vor. Daneben kann das in dem Metallcarbonatgemisch enthaltene Kupfer und Zink auch in Form von Metallcarbonaten verschiedenen Metallsalz(en) in den oben für Verunreinigungen angegebenen Mengen vorliegen.

In dieser bevorzugten Ausführungsform können in dem Metallcarbonatgemisch maximal 15 Gew.-%, bevorzugt maximal 10 Gew.-%, besonders bevorzugt maximal 5 Gew.-%, eines von Kupfer und Zink verschiedenen Metalls in Form eines oder mehrerer Metallcarbonate und/oder in Form eines von Metallcarbonaten verschiedenen Metallsalzes enthalten sein. Bevorzugt ist das von Kupfer und Zink verschiedene Metall ausgewählt unter Co, Ni, Au, Cd, Ru und Fe.

Insbesondere enthält das Metallcarbonatgemisch, abgesehen von den oben erwähnten Verunreinigungen, keine weiteren von Kupfer und Zink verschiedene Metalle.

Falls das in der metallcarbonathaltigen Masse enthaltene Metallcarbonatgemisch Kupfer- und Zink enthält, liegt das Molverhältnis von Kupfer und Zink im Metallcarbonatgemisch bevorzugt im Bereich von 2 : 1 bis 1 : 1,5, besonders bevorzugt im Bereich von 1,7 : 1 bis 1 : 1,2 und insbesondere im Bereich von 1,5 : 1 bis 1 : 1. Dementsprechend enthält die in Schritt a) bereitgestellte metallcarbonathaltige Masse in einer weiteren besonders bevorzugten Ausführungsform des erfindungsgemäßen Verfahrens, bezogen auf das Gesamtgewicht der metallcarbonathaltigen Masse,

- 70 Gew.-% oder mehr eines Metallcarbonatgemisches, enthaltend 2 bis 10 Metallcarbonate von 2 bis 10 verschiedenen Metallen (M),
- 10 bis 25 Gew.-% metallisches Kupfer und
- 0,5 bis 5 Gew.-% Tablettierhilfsmittel,

wobei das in der metallcarbonathaltigen Masse enthaltene Metallcarbonatgemisch, Kupfer und Zink enthält, und wobei das Molverhältnis von Kupfer und Zink im Metallcarbonatgemisch im Bereich von 2 : 1 bis 1 : 1,5 liegt.

Erfindungsgemäß enthält die in Schritt a) bereitgestellte metallcarbonathaltige Masse zusätzlich 10 bis 25 Gew.-%, bevorzugt 10 bis 20 Gew.-%, insbesondere 10 bis 15 Gew.-% metallisches Kupfer. Üblicherweise wird das metallische Kupfer in einer zum Mischen und Pressen geeigneten Form, wie beispielsweise Kupferpulver oder Kupferblättchen, zur metallcarbonathaltigen Masse zugegeben.

Bevorzugt handelt es sich bei dem metallischen Kupfer um Kupferpulver und/oder Kupferblättchen.

Des Weiteren enthält die in Schritt a) bereitgestellte metallcarbonathaltige Masse zusätzlich 0,5 bis 5 Gew.-%, bevorzugt 0,5 bis 4 Gew.-%, insbesondere 1 bis 3 Gew.-% Tablettierhilfsmittel.

Bei dem Tablettierhilfsmittel handelt es sich üblicherweise um Verbindungen, die die bei der Formgebung auftretende Reibung reduzieren, wie beispielsweise Öle, Stearate, Graphit, Bornitrid oder Molybdändisulfid.

Bevorzugt ist das Tablettierhilfsmittel ausgewählt unter Graphit, Bornitrid, Molybdändisulfid und Mischungen davon.

In einer besonders bevorzugten Ausführungsform des erfindungsgemäßen Verfahrens zur Herstellung eines Katalysatorformkörpers, wird in Schritt a) eine metallcarbonathaltige Masse bereitgestellt, die, bezogen auf das Gesamtgewicht der metallcarbonathaltigen Masse,

- 82 bis 89 Gew.-% eines Metallcarbonatgemisches, enthaltend 2 bis 5 Metallcarbonate von 2 bis 5 verschiedenen Metallen (M),
- 10 bis 15 Gew.-% metallisches Kupfer und
- 1 bis 3 Gew.-% Tablettierhilfsmittel,

enthält.

In einer weiteren besonders bevorzugten Ausführungsform des erfindungsgemäßen Verfahrens zur Herstellung eines Katalysatorformkörpers, wird in Schritt a) eine metallcarbonathaltige Masse bereitgestellt, die, bezogen auf das Gesamtgewicht der metallcarbonathaltigen Masse,

- 82 bis 89 Gew.-% eines Metallcarbonatgemisches, enthaltend 2 bis 5 Metallcarbonate von 2 bis 5 verschiedenen Metallen (M),
- 10 bis 15 Gew.-% metallisches Kupfer und
- 1 bis 3 Gew.-% Tablettierhilfsmittel,

enthält,
wobei das in der metallcarbonathaltigen Masse enthaltene Metallcarbonatgemisch, bezogen auf das Gesamtgewicht des Metallcarbonatgemisches,

- 22 bis 36 Gew.-% Kupfer und
- 18 bis 33 Gew.-% Zink

enthält.

In einer speziellen Ausführungsform des erfindungsgemäßen Verfahrens zur Herstellung eines Katalysatorformkörpers, wird in Schritt a) eine metallcarbonathaltige Masse bereitgestellt, die, bezogen auf das Gesamtgewicht der metallcarbonathaltigen Masse,

- 82 bis 89 Gew.-% eines Metallcarbonatgemisches, enthaltend 40 bis 65 Gew.-% Kupfercarbonat, 35 bis 60 Gew.-% Zinkcarbonat und 0 bis 20 Gew.-% eines von Kupfercarbonat und Zinkcarbonat verschiedenen Metallcarbonats,
- 10 bis 15 Gew.-% metallisches Kupfer und
- 1 bis 3 Gew.-% Tablettierhilfsmittel,

enthält.

In einer weiteren speziellen Ausführungsform des erfindungsgemäßen Verfahrens zur Herstellung eines Katalysatorformkörpers, wird in Schritt a) eine metallcarbonathaltige Masse bereitstellt, die, bezogen auf das Gesamtgewicht der metallcarbonathaltigen Masse,

- 82 bis 89 Gew.-% eines Metallcarbonatgemisches, enthaltend, bezogen auf das Gesamtgewicht des Metallcarbonatgemisches, 22 bis 36 Gew.-% Kupfer, 18 bis 33 Gew.-% Zink und 0 bis 15 Gew.-% eines von Kupfer und Zink verschiedenen Metalls,
- 10 bis 15 Gew.-% metallisches Kupfer und
- 1 bis 3 Gew.-% Tablettierhilfsmittel,

enthält.

In einer ganz speziellen Ausführungsform des erfindungsgemäßen Verfahrens zur Herstellung eines Katalysatorformkörpers, wird in Schritt a) eine metallcarbonathaltige Masse bereitstellt, die, bezogen auf das Gesamtgewicht der metallcarbonathaltigen Masse,

- 82 bis 89 Gew.-% eines Metallcarbonatgemisches, bestehend aus 40 bis 65 Gew.-% Kupfercarbonat und 35 bis 60 Gew.-% Zinkcarbonat,
- 10 bis 15 Gew.-% metallisches Kupfer und
- 1 bis 3 Gew.-% Tablettierhilfsmittel,

enthält.

In einer weiteren ganz speziellen Ausführungsform des erfindungsgemäßen Verfahrens zur Herstellung eines Katalysatorformkörpers, wird in Schritt a) eine metallcarbonathaltige Masse bereitstellt, die, bezogen auf das Gesamtgewicht der metallcarbonathaltigen Masse,

- 82 bis 89 Gew.-% eines Metallcarbonatgemisches, enthaltend, bezogen auf das Gesamtgewicht des Metallcarbonatgemisches, 22 bis 36 Gew.-% Kupfer und 18 bis 33 Gew.-% Zink,
- 10 bis 15 Gew.-% metallisches Kupfer und
- 1 bis 3 Gew.-% Tablettierhilfsmittel,

enthält,
wobei das in der metallcarbonathaltigen Masse enthaltene Metallcarbonatgemisch, abgesehen von Verunreinigungen, keine weiteren von Kupfer und Zink verschiedene Metalle enthält.

Erfindungsgemäß umfasst die Bereitstellung der metallcarbonathaltigen Masse in Schritt a) die folgenden Schritte

a1) Bereitstellung eines pulverförmigen Metallcarbonatgemisches, enthaltend zwei oder mehr als zwei Metallcarbonate von zwei oder mehr als zwei verschiedenen Metallen (M), und

a2) Zugabe des metallischen Kupfers und des Tablettierhilfsmittels zu dem in Schritt a1) bereitgestellten pulverförmigen Metallcarbonatgemisch. Erfindungsgemäß erfolgt die Bereitstellung des pulverförmigen Metallcarbonatgemisches nach üblichen im Stand der Technik bekannten Verfahren zur Herstellung von Metallcarbonaten und/oder Metallcarbonatgemischen.

Bevorzugt erfolgt die Herstellung des Metallcarbonatgemisches analog zu dem in DE 102014004413 beschrieben Verfahren.

Hiernach wird üblicherweise so vorgegangen, dass zunächst ein Metallcarbonat-haltiger Niederschlag durch Vereinigen einer Lösung A und einer Lösung B gebildet wird. Der Niederschlag wird danach von dem flüssigen Anteil der vereinigten Lösungen abgetrennt und gewaschen. Anschließend wird der abgetrennte Niederschlag durch Erwärmen auf eine Temperatur im Bereich von 75°C bis 140°C getrocknet.

Die Lösung A wird hergestellt, indem zwei oder mehr als zwei Metallverbindungen von zwei oder mehr als zwei verschiedenen Metallen (M) in einem Behälter in einem geeigneten Lösungsmittel gelöst werden.

Alternativ können die zwei oder mehr als zwei Metallverbindungen von zwei oder mehr als zwei verschiedenen Metallen (M) in mehreren Behältern gelöst werden und die daraus erhältlichen Lösungen zu einer Lösung A vereinigt werden.

Die Lösung B wird hergestellt, indem eine Carbonatverbindung in einem geeigneten Lösungsmittel gelöst wird.

In diesem Zusammenhang schließen die hier verwendeten Ausdrücke "Lösungen A" und "Lösungen B" sowohl Lösungen als auch Suspensionen und Aufschlämmungen ein, wobei Lösungen bevorzugt sind.

Vorzugsweise ist das Lösungsmittel Wasser. Dem Wasser kann eine Säure oder Base zugesetzt werden, um das Lösen der Verbindungen zu unterstützen. Das Wasser kann beispielsweise einen neutralen pH-Wert von etwa 7, einen sauren pH-Wert von etwa 0 bis kleiner als 7, oder einen basischen pH-Wert von größer als 7 bis etwa 14 aufweisen.

Wie dem Fachmann bekannt ist, wird ein zum Lösen der Verbindungen geeigneter pH-Wert in Abhängigkeit von der zu lösenden Verbindung gewählt. Üblicherweise weist das Wasser einen pH-Wert im Bereich von 4 bis 10, vorzugsweise 5 bis 9 auf.

Als Metallverbindungen können prinzipiell sowohl die jeweiligen Metalle in metallischer Form als auch vorzugsweise alle in Wasser, Säuren oder Laugen gut löslichen Verbindungen der jeweiligen Metalle (M), wie oben definiert, verwendet werden.

Besonders bevorzugt sind die zur Herstellung der Lösung A eingesetzten Metallverbindungen von zwei oder mehr als zwei verschiedenen Metallen (M) ausgewählt unter den Nitraten, Carbonaten, Hydroxycarbonaten, Hydrogencarbonaten, Halogeniden, wie beispielsweise die Chloride, Bromide und/oder Iodide, Oxiden, Hydroxiden, Acetaten, Aminkomplexen, Phosphaten, Sulfiten und/oder Sulfaten der Metalle (M), wie oben definiert.

Wenn Oxide der Metalle (M), wie beispielsweise Kupferoxid und/oder Zinkoxid, zur Herstellung der wässrigen Lösungen verwendet werden, dann werden diese vorzugsweise durch Zugabe einer geeigneten Mineralsäure zum Teil oder vollständig gelöst. Die Mineralsäure ist vorzugsweise ausgewählt aus $HNO_3$, HCl, $H_2SO_4$ und Mischungen davon. Falls als Metalloxid Kupferoxid eingesetzt wird kann das Kupfer in Kupferoxid in einer oder in mehreren verschiedenen Oxidationsstufen, wie Kupfer(I)-oxid, Kupfer(II)-oxid und Mischungen davon vorliegen. Wenn die Metalle, wie beispielsweise Kupfer und/oder Zink, selbst zur Herstellung der wässrigen Lösung(en), Suspension(en) oder Aufschlämmung(en) verwendet werden, dann werden diese vorzugsweise durch Zugabe geeigneter Säuren oder Laugen zum Teil oder vollständig gelöst. Die Auflösung der Metalle kann beispielsweise in anorganischen Säuren oder Laugen erfolgen.

Ganz besonders bevorzugt sind die zur Herstellung der Lösung A eingesetzten Metallverbindungen von zwei oder mehr als zwei verschiedenen Metallen (M) ausgewählt unter den Nitraten, Carbonaten, Hydroxycarbonaten, Hydrogencarbonaten, Chloriden, Bromiden, Hydroxiden und Sulfaten von Co, Ni, Cu, Au, Zn, Cd, Ru und Fe.

Insbesondere sind die zur Herstellung der Lösung A eingesetzten Metallverbindungen von zwei oder mehr als zwei verschiedenen Metallen (M) ausgewählt unter den Nitraten, Carbonaten, Hydroxycarbonaten, Hydrogencarbonaten, Chloriden, und Sulfaten von Ni, Cu, Au und Zn.

Falls zur Herstellung der Lösung A als Metallverbindung eine Kupferverbindung eingesetzt wird, ist diese bevorzugt ausgewählt unter Kupferoxid ($Cu_2O$ und/oder CuO), Kupfernitrat, Kupferchlorid, Kupfercarbonat bzw. Kupferhydroxycarbonat, wie oben definiert, Cu-Aminkomplexen (wie Kupfertetraminkomplexen ($[Cu(NH_3)_4]^{2+}$) oder Kupferhexaminkomplexen ($[Cu(NH_3)_6]^{2+}$), die beispielsweise als Chlorid, Hydroxid oder Sulfat eingesetzt werden können), Kupferacetat und Kupfersulfat, besonders bevorzugt unter Kupfernitrat, Kupferchlorid und Kupfersulfat. Alternativ kann auch Kupfermetall in oxidierenden Säuren, wie Salpetersäure ($HNO_3$) gelöst werden.

Falls zur Herstellung der Lösung A als Metallverbindung eine Zinkverbindung eingesetzt wird, ist diese bevorzugt ausgewählt unter Zinknitrat, Zinksulfat, Zinkchlorid, Zinkcarbonat, Zinkhydroxid, Zinksulfit, Zinkacetat und Zinkphosphat, insbesondere unter Zinknitrat, Zinkchlorid und Zinksulfat. Alternativ kann auch Zinkmetall oder ZnO in Säuren, wie Salzsäure (HCl) oder Salpetersäure ($HNO_3$), oder in Laugen, wie Natronlauge (NaOH) oder Kalilauge (KOH), gelöst werden.

Speziell sind die zur Herstellung der Lösung A eingesetzten Metallverbindungen Kupfersulfat und Zinksulfat.

Die zur Herstellung der Lösung B eingesetzten Carbonatverbindungen sind vorzugsweise ausgewählt unter Alkalicarbonaten, wie Lithium-, Natrium-, Kalium-, Rubidium- oder Cäsium-Carbonat, Erdalkalicarbonaten, wie Magnesium-, Kalzium-, Strontium- oder Barium-Carbonat, Ammoniumcarbonat oder Mischungen davon. Ebenso können gleichzeitig mit oder anstelle der Carbonate die entsprechenden Hydrogencarbonate oder beliebige Mischungen aus Carbonaten und Hydrogencarbonaten verwendet werden.

Vorzugsweise werden Alkalicarbonate, Ammoniumcarbonate, Alkalihydrogencarbonate, Ammoniumhydrogencarbonate oder Mischungen davon, besonders bevorzugt Alkalicarbonate und/oder -hydrogencarbonate verwendet.

Bevorzugte Alkalicarbonate sind Natrium- und Kaliumcarbonat, insbesondere Natriumcarbonat. Bevorzugte Alkalihydrogencarbonate sind Natrium- und Kaliumhydrogencarbonat, insbesondere Natriumhydrogencarbonat. Besonders bevorzugt ist die Verwendung von Natriumcarbonat und/oder Natriumhydrogencarbonat.

Das Vereinigen erfolgt üblicherweise indem die Lösung A und Lösung B gleichzeitig in einen gemeinsamen Behälter, wie beispielsweise einen Fällbehälter, gegeben werden. Dabei werden die beiden Lösungen vorzugsweise kontinuierlich in das Reaktionsvolumen eines Fällmischers eingeleitet. In einer weiteren Ausführungsform kann das Vereinigen auch erfolgen, indem die Lösung A oder Lösung B zu der, beispielsweise in einem Behälter, wie einem Fällbehälter, vorgelegten zugehörigen anderen Lösung B oder Lösung A zudosiert wird. In einer bevorzugten Ausführungsform erfolgt das Vereinigen der Lösungen durch Zudosieren der Lösung A zu der in einem Fällbehälter vorgelegten carbonathaltigen Lösung B.

Gegebenenfalls kann die Lösung A vor dem Vereinigen auf eine Temperatur von 20°C oder mehr, wie zum Beispiel auf eine Temperatur im Bereich von 25°C bis 90°C erwärmt und dabei vorzugsweise gerührt werden. Vorzugsweise wird die Lösung A vor dem Vereinigen nicht erwärmt.

Gegebenenfalls kann die Lösung B vor dem Vereinigen auf eine Temperatur von 20°C oder mehr, wie zum Beispiel auf eine Temperatur im Bereich von 25°C bis 90°C erwärmt und dabei vorzugsweise gerührt werden. Vorzugsweise wird die Lösung B vor dem Vereinigen nicht erwärmt.

Beim Vereinigen der Lösung A und Lösung B bildet sich ein Metallcarbonat-haltiger Niederschlag, der üblicherweise als Suspension vorliegt. Das Vereinigen der Lösungen erfolgt in der Regel in einem gerührten Behälter. Der Behälter wird vorzugsweise mit einem Schrägblattrührer, Propellerrührer oder sonstigen handelsüblichen Rührern gerührt.

Die nach der Vereinigung der Lösung A und Lösung B erhaltene Suspension aus dem Metallcarbonat-haltigen Niederschlag wird üblicherweise auf eine Temperatur von 20°C oder mehr, wie zum Beispiel auf eine Temperatur im Bereich von 25°C bis 90°C, insbesondere im Bereich von 30°C bis 70°C erwärmt und für einige Minuten bis einige Stunden, wie zum Beispiel für 5 Minuten bis 5 Stunden, insbesondere für 20 Minuten bis 2 Stunden, gerührt.

Der Metallcarbonat-haltige Niederschlag wird vorzugsweise durch Filtration abgetrennt. Alternativ dazu kann der Niederschlag auch durch Dekantieren oder Zentrifugieren abgetrennt werden.

Der abgetrennte Niederschlag wird anschließend einem oder mehreren Waschschritten unterzogen. Dabei kann die niederschlagshaltige Lösungsmischung zuerst durch Verwendung einer Filterpresse vom Präzipitat abgetrennt und anschließend das Material in der Filterpresse mit Wasser durchströmt und dadurch gewaschen werden. Alternativ kann der abgetrennte Niederschlag nach dem Abtrennen der niederschlagshaltigen Lösungsmischung durch Filtrieren, Dekantieren oder Zentrifugieren in einem Behälter aufgeschlämmt und anschließend erneut mit Hilfe einer Filterpresse, einer Zentrifuge oder eines Dekanters von der flüssigen Phase getrennt werden. Dieser Vorgang wird in der Regel ein oder mehrere Male bis zum Erreichen einer bestimmten Leitfähigkeit des Filtrats durchgeführt.

Der abgetrennte und gewaschene Niederschlag wird anschließend einer Trocknung unterzogen. Die Trocknung erfolgt durch Erwärmen des Niederschlags auf eine Temperatur im Bereich von 75°C bis 140°C, vorzugsweise in einem Bereich von 90°C bis 130°C. Die Trocknung kann beispielsweise durch Sprühtrocknung oder durch Trocknung in einem Trockenschrank erfolgen.

Die Trocknung kann bei Umgebungsdruck oder vermindertem Druck erfolgen. Bevorzugt erfolgt die Trocknung bei Umgebungsdruck.

Das so erhaltene Metallcarbonatgemisch wird anschließend zu einem Pulver zermahlen, falls dieses nicht schon durch die Art der Trocknung in geeigneter Pulverform vorliegt, wie dies üblicherweise bei der Sprühtrocknung der Fall ist, und gegebenenfalls auf eine gewünschte Korngröße, beispielsweise einer Korngröße im Bereich von 0,1 bis 2 mm, bevorzugt im Bereich von 0,3 bis 1,6 mm gesiebt. Gegebenenfalls wird dem Metallcarbonatgemisch zusätzlich eine oder mehrere von Metallcarbonaten verschiedene Metallverbindung(en), wie oben definiert, in einer zum Mischen und Pressen geeigneten Form, beispielsweise in Form eines Pulvers, zugesetzt.

Zu dem pulverförmigen Metallcarbonatgemisch wird dann metallisches Kupfer in einer zum Mischen und Pressen geeigneten Form, wie beispielsweise in Form von Kupferpulver oder Kupferblättchen, sowie das Tablettierhilfsmittel, ebenfalls in einer zum Mischen und Pressen geeigneten Form, beispielsweise in Form eines Pulvers, in den oben angegebenen Mengen zugegeben (Schritt a2).

### Zu Schritt b) des Verfahrens

Die so in Schritt a) bereitgestellte metallcarbonathaltige Masse wird anschließend zu einem Formkörper geformt.

Hierzu können alle dem Fachmann bekannten Formgebungsverfahren verwendet werden. Bevorzugt erfolgt die Formgebung durch Pressen bzw. Tablettieren der in Schritt a) bereitgestellten metallcarbonathaltigen Masse zu den gewünschten Formkörpern.

Bevorzugt handelt es sich bei den Formkörpern um rieselfähige Teilchen mit einem maximalen Durchmesser im Bereich von 1 bis 20 mm, wie beispielsweise Granulat oder Tabletten. Besonders bevorzugt handelt es sich bei den Formkörpern um Tabletten mit einem Durchmesser im Bereich von 1 bis 10 mm und mit einer Höhe im Bereich von 1 bis 10 mm, bevorzugt mit einem Durchmesser im Bereich von 1,5 bis 5 mm und mit einer Höhe im Bereich von 1,5 bis 5 mm.

Die Tablettierung wird vorzugsweise mit einer Tablettenpresse, wie beispielsweise einer Kilian E150 Plus Tablettenpresse, durchgeführt.

Nach der Tablettierung weisen die Tabletten üblicherweise eine Seitendruckfestigkeit, gemessen gemäß DIN EN 1094-5, von wenigstens 10 N, bevorzugt von wenigstens 20 N, insbesondere von wenigstens 25 N, speziell von 25 bis 110 N, ganz speziell von 25 bis 90 N, und ein Schüttgewicht im Bereich von 1,1 bis 2,2 g/ml, bevorzugt im Bereich von 1,2 bis 2,0 g/ml, insbesondere im Bereich von 1,3 bis 1,9 g/ml, auf.

Vorzugsweise weisen die durch das Tablettieren hergestellten Tabletten einen Durchmesser im Bereich von 1,5 bis 5 mm, eine Höhe im Bereich von 1,5 bis 5 mm und eine Seitendruckfestigkeit von wenigstens 25 N, speziell von 25 bis 110 N, ganz speziell von 25 bis 90 N, auf.

Nach der Formgebung und vor der Aktivierung in Schritt c) enthalten die Katalysatorformkörper einen Carbonatgehalt im Bereich von 15 bis 45 Gew.-%.

Bevorzugt enthalten die Katalysatorformkörper nach der Formgebung und vor der Aktivierung in Schritt c) einen Carbonatgehalt im Bereich von 15 bis 45 Gew.-%, besonders bevorzugt von 17 bis 40 Gew.-%, insbesondere von 20 bis 35 Gew.-%.

Der Carbonatgehalt, insbesondere des Katalysatorformkörpers bzw. der metallcarbonathaltigen Masse, wird hierfür in Analogie zu der in F. Ehrenberger: "Quantitative Organische Elementaranalyse", VCN Verlagsgesellschaft mbH, Weinheim; Ausgabe 1991; ISBN: 3-527-28056-1, Seiten 225 ff. beschriebenen Methode bestimmt.

Hiernach wird der in den jeweiligen Probe des Katalysatorformkörpers bzw. der metallcarbonathaltigen Masse enthaltene anorganische Kohlenstoff, der üblicherweise in Form von Carbonat-, Hydroxycarbonat- und/oder Hydrogencarbonat-Ionen vorliegt, unter geringer Wärmezufuhr mit verdünnter Phosphorsäure in Kohlensäure überführt, die zu $CO_2$ und $H_2O$ zerfällt. Das so freigesetzte $CO_2$ wird mit einem Inertgasstrom aus der Probe ausgetrieben. In einer nachgeschalteten Gaswäsche werden störende Komponenten entfernt. Der Gehalt an $CO_2$ in dem Inertgasstrom wird anschließend mittels Infrarotspektroskopischer Messung quantifiziert. Bei dieser Methode liegt die kleinste Menge an anorganischem Kohlenstoff, die sich noch zuverlässig in einer Probe bestimmen lässt, bei etwa 1 mg anorganischem Kohlenstoff pro 100 g Probe.

In einer ersten Ausführungsform des erfindungsgemäßen Verfahrens zur Herstellung eines Katalysatorformkörpers wird der in Schritt b) erhaltene Formkörper anschließend direkt einer Aktivierung (Schritt c)) zugeführt.

**Zu Schritt c) des Verfahrens**

Gemäß Schritt c) des erfindungsgemäßen Verfahrens erfolgt die Aktivierung des in Schritt b) erhaltenen Formkörpers bei einer Temperatur im Bereich von 150 bis 250 °C in Gegenwart von Wasserstoff, d.h. in Gegenwart einer reduzierenden Atmosphäre.

Im Rahmen der vorliegenden Anmeldung bedeutet der Ausdruck "in Gegenwart von Wasserstoff", dass die Aktivierung unter einer Wasserstoff-Atmosphäre oder unter einer Wasserstoff-haltigen Atmosphäre durchgeführt wird. Alternativ bedeutet der Ausdruck "in Gegenwart von Wasserstoff" auch, dass die in Schritt b) erhaltenen Formkörper mit Wasserstoff oder einem Wasserstoff-haltigen Gas durchströmt werden.

Bevorzugt wird die Aktivierung derart durchgeführt, dass die in Schritt b) erhaltenen Formkörper mit Wasserstoff oder einem Wasserstoff-haltigen Gas, bevorzugt mit einem Wasserstoff-haltigen Gas, durchströmt werden.

Bei dem Wasserstoff-haltigen Gas handelt es sich im Rahmen der vorliegenden Erfindung um ein Gasgemisch, das aus einem oder mehreren inerten Gas(en) und 0,1 Vol.-% bis 99,9 Vol.-% Wasserstoff besteht.

Falls die in Schritt b) erhaltenen Formkörper zur Aktivierung mit einem Wasserstoff-haltigen Gas durchströmt werden, kann die Konzentration an Wasserstoff in dem Wasserstoff-haltigen Gas konstant gehalten oder über den Verlauf der Aktivierung langsam erhöht werden, beispielsweise von etwa 0,1 Vol.-% auf etwa 99,9 Vol.-%. Beispielsweise kann der Formkörper zur Aktivierung mit einem Gemisch, bestehend aus Wasserstoff und einem oder mehreren inerten Gas(en), durchströmt werden, wobei das Verhältnis von Wasserstoff zu dem/den inerten Gas(en) anfangs etwa 0,1 Vol.-% Wasserstoff zu 99,9 Vol.-% Inertgas(en) beträgt. Das Verhältnis von Wasserstoff zu dem/den Inertgas(en) wird dann allmählich erhöht (z. B. kontinuierlich oder schrittweise) bis letztendlich der Katalysatorformkörper beispielsweise mit 99,9 Vol.-% Wasserstoff durchströmt wird, wobei der Katalysatorformkörper zum Schluss auch mit reinem Wasserstoff durchströmt werden kann. Üblicherweise findet dieser Vorgang unter Temperaturkontrolle statt. In diesem Zusammenhang bedeutet der Ausdruck "unter Temperaturkontrolle", dass der Anteil des Wasserstoffs in dem Wasserstoff-haltigen Gas derart erhöht wird, dass die Temperatur des Katalysatorformkörpers 300°C, bevorzugt 250 °C nicht übersteigt, und insbesondere im Bereich von 150 bis 250°C liegt.

Falls die Konzentration des Wasserstoffs in dem Wasserstoff-haltigen Gas während der Aktivierung konstant gehalten wird, handelt es sich bei dem Wasserstoff-haltigen Gas um eine Gasgemisch, das aus einem oder mehreren inerten Gas(en) und wenigstens 5 Vol.-%, bevorzugt wenigstens 10 Vol.-%, insbesondere 10 bis 95 Vol.-% Wasserstoff besteht.

Bevorzugt wird die Konzentration an Wasserstoff in dem Wasserstoff-haltigen Gas über den Verlauf der Aktivierung langsam erhöht, wie oben definiert.

Der im Rahmen der vorliegenden Erfindung verwendete Ausdruck "inertes Gas" bezieht sich auf Gase, welche unter den gegebenen Verfahrensbedingungen keine Reaktionen mit dem Katalysatorformkörper eingehen. Üblicherweise ist das inerte Gas ausgewählt unter Stickstoff und Edelgasen, wie beispielsweise Helium oder Argon, bevorzugt ist das inerte Gas ausgewählt unter Stickstoff und Argon. Insbesondere handelt es sich bei dem inerten Gas um Stickstoff.

Bevorzugt erfolgt die Aktivierung bei einer Temperatur im Bereich von 170°C bis 240°C, insbesondere im Bereich von 190°C bis 210°C.

Die Dauer der Aktivierung ist von der zu reduzierenden Katalysatormenge abhängig und kann daher stark variieren. Beispielsweise erfolgt die Aktivierung einer Katalysatormenge im Bereich von 10 bis 1000 g über einen Zeitraum von 0,5 bis 2 Stunden. Für Katalysatormengen im Bereich von 10 kg bis 1000 kg erfolgt die Aktivierung beispielsweise über einen Zeitraum von 4 Stunden bis 3 Tagen.

Falls die Aktivierung unter einer Wasserstoff-Atmosphäre oder unter einer Wasserstoff-haltigen Atmosphäre durchgeführt wird, kann die Aktivierung bei Umgebungsdruck oder unter erhöhtem Druck, beispielsweise unter einem Druck im Bereich von 1,1 bis 250 bar, erfolgen.

Bevorzugt wird die Aktivierung bei Umgebungsdruck durchgeführt.

In einer zweiten Ausführungsform des erfindungsgemäßen Verfahrens zur Herstellung eines Katalysatorformkörpers wird der in Schritt b) erhaltenen Formkörper zunächst in einem Schritt c1) einer ersten thermischen Behandlung bei einer Temperatur im Bereich von 150 bis 350 °C in Abwesenheit von Wasserstoff unterworfen. Der so thermisch behandelte Formkörper wird anschließend in einem Schritt c2) bei einer Temperatur im Bereich von 150 bis 250 °C in Gegenwart von Wasserstoff aktiviert.

**Zu Schritt c1)**

Die erste thermische Behandlung in Abwesenheit von Wasserstoff erfolgt in der Regel bei einer Temperatur im Bereich von 150 bis 350 °C, bevorzugt im Bereich von 180 bis 310 °C, besonders bevorzugt im Bereich von 200 bis 300 °C insbesondere im Bereich von 200 bis 250 °C.

Erfindungsgemäß erfolgt die thermische Behandlung in Abwesenheit von Wasserstoff. Diese bedeutet, dass die thermische Behandlung unter Luft oder unter einem inerten Gas, wie oben definiert, oder unter einem Gemisch aus Luft mit einem inerten Gas erfolgt. Bevorzugt erfolgt die thermische Behandlung unter Luft.

Die nach der thermischen Behandlung erhaltenen Katalysatorformkörper, insbesondere die Katalysator-Tabletten, weisen üblicherweise eine Seitendruckfestigkeit, gemessen gemäß DIN EN 1094-5, von wenigstens 5 N, bevorzugt von wenigstens 10 N, insbesondere von wenigstens 15 N, speziell von 15 bis 90 N, ganz speziell von 15 bis 80 N, und ein Schüttgewicht im Bereich von 0,8 bis 1,6 g/ml, bevorzugt im Bereich von 0,9 bis 1,5 g/ml, insbesondere im Bereich von 1,0 bis 1,4 g/ml, auf.

Vorzugsweise weisen die nach der thermischen Behandlung erhaltenen Katalysatorformkörper, insbesondere die Katalysator-Tabletten, einen Durchmesser im Bereich von 1,5 bis 5 mm, eine Höhe im Bereich von 1,5 bis 5 mm und eine Seitendruckfestigkeit von wenigstens 15 N, speziell von 15 bis 90 N, ganz speziell von 15 bis 80 N, auf. Die nach der thermischen Behandlung und vor der Aktivierung in Schritt c2) erhaltenen Katalysatorformkörper dieser zweiten Ausführungsform weisen üblicherweise einen Carbonatgehalt im Bereich von 0,1 bis 2,5 Gew.-% auf.

Bevorzugt weisen die nach der thermischen Behandlung und vor der Aktivierung in Schritt c2) erhaltenen Katalysatorformkörper dieser zweiten Ausführungsform einen Carbonatgehalt im Bereich von 0,2 bis 2,3 Gew.-%, insbesondere von 0,4 bis 2,1 Gew.-% auf.

**Zu Schritt c2)**

Gemäß der zweiten Ausführungsform des erfindungsgemäßen Verfahrens werden die thermisch behandelten Formkörper anschließend in einem Schritt c2) bei einer Temperatur im Bereich von 150 bis 250 °C in Gegenwart von Wasserstoff aktiviert.

Die Aktivierung in Schritt c2) erfolgt analog zum Schritt c) der ersten Ausführungsform. Hinsichtlich der Durchführung des Schrittes c2) wird auf die zuvor unter Schritt c) gemachten Ausführungen Bezug genommen.

In einer bevorzugten Ausführungsform des erfindungsgemäßen Verfahrens zur Herstellung eines Katalysatorformkörpers wird der Katalysatorformkörper im Anschluss an die Aktivierung in Schritt c) oder c2) bei einer Temperatur von 60°C oder weniger in Gegenwart eines sauerstoffhaltigen Gasgemisches, insbesondere von Luft, passiviert.

In diesem Zusammenhang bezieht sich der Begriff "sauerstoffhaltiges Gasgemisch" auf Luft sowie auf Gasgemische aus Sauerstoff oder Luft mit einem inerten Gas.

Bei der Passivierung wird in den Reduktionsreaktor Luft oder ein Gemisch aus Sauerstoff oder Luft und einem inerten Gas, wie Argon oder Stickstoff, zudosiert.

Falls die Passivierung in Gegenwart eines Gemisches aus Sauerstoff oder Luft und einem inerten Gas, wie Argon oder Stickstoff, erfolgt, kann die Konzentration an Sauerstoff in dem Gemisch konstant gehalten oder über den Verlauf der Passivierung langsam erhöht werden, beispielsweise von etwa 0,04 Vol.-% auf etwa 21 Vol.-%. Beispielsweise kann ein Gemisch aus Luft und Inertgas zudosiert werden, wobei das Verhältnis von Luft zu Inertgas anfangs etwa 0,2 Vol.-% Luft zu 99,8 Vol.-% Inertgas beträgt. Das Verhältnis von Luft zu Inertgas wird dann allmählich erhöht (z. B. kontinuierlich oder schrittweise) bis letztendlich beispielsweise 100 Vol.-% Luft zudosiert wird (was einer Sauerstoffkonzentration von etwa 21 Vol.-% entspricht).

Bevorzugt erfolgt die Passivierung in Gegenwart von Luft. Üblicherweise erfolgt die Passivierung bei einer Temperatur von 60°C oder weniger.

Bevorzugt erfolgt die Passivierung bei einer Temperatur im Bereich von 5 bis 55 °C, besonders bevorzugt im Bereich von 10 bis 50 °C, insbesondere im Bereich von 15 bis 45 °C.

Falls die Aktivierung des in Schritt b) erhaltenen Formkörpers in-situ im Hydrierreaktor durchgeführt wird, kann in der Regel auf eine Passivierung verzichtet werden.

Die durch das oben beschriebene erfindungsgemäße Herstellungsverfahren erhaltenen Katalysatorformkörper weisen gegenüber herkömmlichen Katalysatorformkörpern, die aus demselben Metallcarbonatgemisch aber ohne die Zugabe von metallischem Kupfer hergestellt wurden, nicht nur eine deutlich erhöhte Stabilität sondern sogar eine erhöhte Hydrieraktivität auf.

Die Beschreibung offenbart Katalysatorformkörper, die durch das oben beschriebene Herstellungsverfahren erhältlich sind.

Die nach dem oben beschriebenen erfindungsgemäßen Herstellungsverfahren, d.h. nach der Aktivierung gemäß einem der Schritte c) oder c2) und gegebenenfalls nachfolgender Passivierung, erhaltenen Katalysatorformkörper, insbesondere die Katalysator-Tabletten, weisen üblicherweise eine Seitendruckfestigkeit, gemessen gemäß DIN EN 1094-5, von wenigstens 5 N, bevorzugt von wenigstens 10 N, insbesondere von wenigstens 15 N, speziell von 15 bis 60, ganz speziell von 15 bis 50 N, und ein Schüttgewicht im Bereich von 0,8 bis 1,6 g/ml, bevorzugt im Bereich von 0,9 bis 1,5 g/ml, insbesondere im Bereich von 1,0 bis 1,4 g/ml, auf.

Vorzugsweise weisen die nach dem oben beschriebenen erfindungsgemäßen Herstellungsverfahren, d.h. nach der Aktivierung gemäß einem der Schritte c) oder c2) und gegebenenfalls nachfolgender Passivierung, erhaltenen Katalysatorformkörper, insbesondere die Katalysator-Tabletten, einen Durchmesser im Bereich von 1,5 bis 5 mm, eine Höhe im Bereich von 1,5 bis 5 mm und eine Seitendruckfestigkeit von wenigstens 15 N, speziell von 15 bis 60, ganz speziell von 15 bis 50 N, auf.

## Hydrierung

Aufgrund ihrer hohen Hydrieraktivität eignen sich die erfindungsgemäß hergestellten Katalysatorformkörper in vorteilhafter Weise für die Verwendung in Hydrierreaktionen. Bevorzugt eignen sich die erfindungsgemäß hergestellten Katalysatorformkörper zur Hydrierung einer eine oder mehr als eine Carbonylgruppe(n) aufweisenden organischen Verbindung.

Bei der Hydrierung einer eine oder mehr als eine Carbonylgruppe(n) aufweisenden organischen Verbindung handelt es sich beispielsweise um die Hydrierung von Estern, insbesondere Fettsäureestern, die Hydrierung von Diestern (insbesondere Maleinsäurediestern) zu Diolen, die Hydrierung von Zuckern zu Polyolen, die Hydrierung eines Aldehyds, die Hydrierung eines Amids, die Hydrierung einer Fettsäure (z. B. durch Veresterung und anschließender Hydrogenolyse), die selektive Hydrierung eines Fettes, die selektive Hydrierung eines Öls, die Hydrierung eines nitroaromatischen Kohlenwasserstoffs, die Hydrierung eines Ketons, die Hydrierung von Furfural und die Hydrierung von Kohlenmonoxid oder Kohlendioxid zu Methanol.

Dementsprechend offenbart die Beschreibung ein Verfahren zur Hydrierung einer eine oder mehr als eine Carbonylgruppe(n) aufweisenden organischen Verbindung, bei dem die organische Verbindung in Gegenwart von Wasserstoff mit einem Katalysatorformkörper, erhältlich durch das oben beschriebene Verfahren, in Kontakt gebracht wird.

Bei der eine oder mehr als eine Carbonylgruppe(n) aufweisenden organischen Verbindung handelt es sich bevorzugt um Aldehyde, Ketone, Carbonsäuren, wie Mono-, Di-, Tri- und Tetra-Carbonsäuren und Ester-Verbindungen, wie Mono-, Di-, Tri- und TetraEster, besonders bevorzugt um Ester-Verbindungen, wie Mono-, Di-, Tri- und TetraEster.

Insbesondere handelt es sich bei der eine oder mehr als eine Carbonylgruppe(n) aufweisenden organischen Verbindung um $C_8$-$C_{24}$-Fettsäurealkylester, speziell um $C_8$-$C_{24}$-Fettsäuremethylester, ganz speziell um $C_{10}$-$C_{18}$-Fettsäuremethylester.

Die in der Hydrierung eingesetzten eine oder mehr als eine Carbonylgruppe(n) aufweisenden organischen Verbindungen, insbesondere die $C_8$-$C_{24}$-Fettsäurealkylester, können als einzelne Verbindungen oder als Gemische, insbesondere als $C_8$-$C_{24}$-Fettsäurealkylestergemische, vorliegen. Falls in der Hydrierung Ester, insbesondere ein $C_8$-$C_{24}$-Fettsäurealkylester, oder Gemische von Estern, insbesondere Gemische von $C_8$-$C_{24}$-Fettsäurealkylestern, eingesetzt werden, können diese zusätzlich die entsprechende(n) Carbonsäure(n), insbesondere die entsprechende(n) $C_8$-$C_{24}$-Fettsäure(n), enthalten. Bevorzugt enthalten die in der Hydrierung eingesetzten Ester, insbesondere die $C_8$-$C_{24}$-Fettsäurealkylester, keine entsprechende(n) Carbonsäure(n). Die Hydrierung kann analog bekannter Hydrierverfahren zur katalytischen Hydrierung von Carbonylgruppen-aufweisenden organischen Verbindungen durchgeführt werden. Hierbei wird die zu hydrierende eine oder mehr als eine Carbonylgruppe(n) aufweisende organische Verbindung in einem oder mehreren Hydrierreaktoren, bevorzugt in einem Hydrierreaktor, üblicherweise in flüssiger oder in gasförmiger Form, bevorzugt in flüssiger Form, in Gegenwart von Wasserstoff mit den erfindungsgemäß hergestellten Katalysatorformkörpern in Kontakt gebracht. Die flüssige Phase kann dabei über eine Flüssigbett oder Festbett, bestehend aus den erfindungsgemäß hergestellten Katalysatorformkörpern, geleitet werden. Bevorzugt wird die flüssige Phase abwärts oder aufwärts über ein Festbett, bestehend aus den erfindungsgemäß hergestellten Katalysatorformkörpern, geleitet. Die katalytische Hydrierung kann kontinuierlich oder chargenweise durchgeführt werden. Als Hydrierreaktoren können alle konventionellen zur Hydrierung von organischen Verbindungen geeigneten im Stand der Technik bekannten Reaktoren, wie zum Beispiel einem Rührreaktor, eingesetzt werden.

Die Hydrierung erfolgt üblicherweise unter erhöhtem Wasserstoff-Druck. Bevorzugt erfolgt die Hydrierung bei einem Wasserstoff-Druck im Bereich von 5 bis 400 bar, insbesondere von 10 bis 300 bar.

Die Hydrierung erfolgt üblicherweise bei einer Temperatur im Bereich von 30 bis 350°C, insbesondere im Bereich von 50 bis 300°C.

**Beispiele**

In den nachfolgenden Beispielen werden folgende Abkürzungen verwendet:

Soda steht für Natriumcarbonat.
SDF steht für Seitendruckfestigkeit.
SG steht für Schüttgewicht.
red/pass steht für reduziert und passiviert.

Bei den Beispielen 1, 4.1 und 4.2 handelt es sich um (nicht erfindungsgemäße) Vergleichsbeispiele. Die übrigen Beispiele sind erfindungsgemäß.

**Beispiel 1 (zum Vergleich):**

Herstellung von Katalysatortabletten <u>ohne</u> Zusatz von Kupfer

*Herstellung einer Metallcarbonatmischung*

Eine Mischung aus 12 kg einer 20 Gew.-%igen wässrigen Kupfersulfatlösung (1,25 mol $CuSO_4$/kg Lösung) und 10 kg einer 20 Gew.-%igen wässrigen Zinksulfatlösung (1,24 mol $ZnSO_4$/kg Lsg.) wird unter Rühren zu 13,5 kg einer auf 20°C temperierten 20 Gew.-%igen Sodalösung (1,89 mol $Na_2CO_3$/kg Lösung) hinzugefügt, bis sich der pH von anfänglich 12 auf 7,2 erniedrigt. Dann wird die Suspension auf 50°C erhitzt und eine Stunde nachgerührt, der pH sinkt dabei weiter leicht ab bis auf einen pH-Wert von 7,0. Die Suspension wird abgekühlt, filtriert und sulfatfrei (<0,1%) gewaschen. Anschließend erfolgt eine Trocknung bei 120°C im Trockenschrank über Nacht.

Das so erhaltene trockene Cu-Zn-Carbonatgemisch enthält, bezogen auf das Gesamtgewicht des Metallcarbonatgemisches, 29 Gew.-% Cu und 25 Gew.-% Zn.

Der Kupfer- und Zinkgehalt wurde mittels Elementaranalyse bestimmt, unter Einsatz der Atomabsorptionsspektrometrie.

*Tablettierung*

Das trockene Metallcarbonatgemisch wird zu einem Metallcarbonatpulver zermahlen, auf 0,4 bis 1,5 mm gesiebt und mit 2 Gew.-% Graphit vermischt. Danach erfolgt die Verformung zu Tabletten mit 3 mm Durchmesser und 3 mm Höhe. Es resultieren Tablettengrünlinge mit einer Seitendruckfestigkeit von 32 N und einem Schüttgewicht von 1,2 g/ml. Nach Reduktion bei 200°C in einem $H_2$-Strom und anschließender Passivierung bei 30°C unter Luft resultieren Tabletten mit einer auf 9 N reduzierten Seitendruckfestigkeit (SDF) bei einem Schüttgewicht (SG) von 0,95 g/ml und einer auf 2,85 mm geschrumpften Tablettendurchmesser (Katalysator 1). Der Masseverlust beim Reduktionsvorgang beträgt 30 Gew.-%.

**Beispiel 2:** Herstellung von Katalysatortabletten mit Zusatz von 15 Gew.-% Kupfer

Die Herstellung der Metallcarbonatmischung erfolgt analog zu Beispiel 1. Das trockene Metallcarbonatgemisch wird zu einem Metallcarbonatpulver zermahlen, auf 0,4 bis 1,5 mm gesiebt und mit 2 Gew.-% Graphit sowie 15 Gew.-% Kupferpulver (Unicoat Kupfer 3845) vermischt. Danach erfolgt die Verformung zu Tabletten mit 3 mm Durchmesser und 3 mm Höhe. Es resultieren Tablettengrünlinge mit einer Seitendruckfestigkeit von 45 N und einem Schüttgewicht von 1,72 g/ml. Die Tablettengrünlinge weisen einen Carbonatgehalt von 20,4 Gew.-% auf (bestimmt wie in Beispiel 6 beschrieben). Nach Reduktion bei 200°C im $H_2$-Strom und anschließender Passivierung bei 30°C unter Luft resultieren Tabletten mit einer auf 21 N reduzierten Seitendruckfestigkeit (SDF) bei einem Schüttgewicht (SG) von 1,2 g/ml und praktisch unverändertem Tablettendurchmesser von 2,99 mm (Katalysator 2).

**Beispiel 3:** Herstellung von Katalysatortabletten mit Zusatz von 15 Gew.-% Kupfer und Vortemperung (thermische Behandlung)

Die Herstellung der Metallcarbonatmischung erfolgt analog zu Beispiel 1. Das trockene Metallcarbonatgemisch wird zu einem Metallcarbonatpulver zermahlen, auf 0,4 bis 1,5 mm gesiebt und mit 2 Gew.-% Graphit sowie 15 Gew.-% Kupferpulver (Unicoat Kupfer 3845) vermischt. Danach erfolgt die Verformung zu Tabletten mit 3 mm Durchmesser und 3 mm Höhe. Es resultieren Tablettengrünlinge mit einer Seitendruckfestigkeit von 45 N und einem Schüttgewicht von 1,72 g/ml. Die Tablettengrünlinge weisen einen Carbonatgehalt von 20,4 Gew.-% auf (bestimmt wie in Beispiel 6 beschrieben). Die so erhaltenen Tabletten werden dann 2h bei 250°C vorgetempert. Nach Reduktion bei 200°C im $H_2$-Strom und anschließender Passivierung bei 30°C unter Luft resultieren Tabletten mit einer auf 20 N reduzierten Seitendruckfestigkeit (SDF) bei einem Schüttgewicht (SG) von 1,30 g/ml und leicht verringerten Tablettendurchmesser von 2,93 mm (Katalysator 3).

**Beispiele 4.1 bis 4.4:** Herstellung von Katalysatortabletten unter Zusatz unterschiedlicher Mengen Kupfer

Es wurden 4 verschiedene Katalysatortabletten analog zu Beispiel 1 bzw. Beispiel 2 hergestellt, wobei vor der Tablettierung 0, 5, 10 und 15 Gew.-% Kupferpulver zugegeben wurde. Zur besseren Vergleichbarkeit wurden die Maschinenparameter der Tabletten-presse so gewählt, dass alle vier Katalysatortabletten-Muster eine Seitendruckfestigkeit von ca. 30 N (gemessen als Tablettengrünlinge, also das direkt aus der Tablettenpresse herausfallende Produkt) aufweisen.

Alle Katalysatortabletten wurden bei 160°C getrocknet, dann bei 220°C im $H_2$-Strom reduziert und anschließend bei 30°C unter Luft passiviert.

Die physikalischen Eigenschaften der so erhaltenen Katalysatortabletten sind in der nachfolgenden Tabelle 1 zusammengefasst.

Tabelle 1: physikalische Eigenschaften von Katalysatortabletten mit unterschiedlichen Mengen an zugesetztem Kupfer

| Bsp.: | Cu-Zusatz [Gew.-%] | CarbonatGehalt *) [Gew.-%] | SDF Grünlinge [N] | SDF nach Red. [N] | Durchmess er nach Red. [mm] | SG nach Red. [g/ml] |
|---|---|---|---|---|---|---|
| 4.1 **) | 0 | 24,2 | 32 | 9 | 2,85 | 0,95 |
| 4.2 **) | 5 | 22,9 | 32 | 9 | 2,92 | 0,98 |
| 4.3 | 10 | 22,0 | 32 | 17 | 2,94 | 1,08 |
| 4.4 | 15 | 20,9 | 31 | 19 | 2,97 | 1,16 |
| *) Carbonatgehalt der Tablettengrünlinge (das direkt aus der Tablettenpresse herausfallende Produkt) **) Vergleichsbeispiel | | | | | | |

Aus den Werten ist ein deutlicher Effekt hinsichtlich der Härte und des Schüttgewichtes der Katalysatortabletten für einen Kupferzusatz von 10 bis 15 Gew.-% zu erkennen. Durch den Zusatz von Kupfer können deutlich härtere und dichtere Katalysatortabletten hergestellt werden.

**Beispiele 5.1 bis 5.5:** Herstellung von Katalysatortabletten mit Zusatz von 15 Gew.-% Kupfer und Vortemperung (thermische Behandlung) bei unterschiedlichen Temperaturen

Katalysatortabletten mit einem Kupferzusatz von 15 Gew.-% wurden analog Beispiel 4.4 hergestellt, wobei die nach der Tablettierung erhaltenen Katalysatortabletten bei 200, 250, 300 und 350 °C vorgetempert, danach bei 220°C im $H_2$-Strom reduziert (aktiviert) und anschließend bei 30°C unter Luft passiviert wurden.

Die physikalischen Eigenschaften der so erhaltenen Katalysatortabletten sind in der nachfolgenden Tabelle 2 zusammengefasst.

Tabelle 2: physikalische Eigenschaften von Katalysatortabletten mit Vortemperung thermische Behandlung) bei unterschiedlichen Temperaturen.

| Bsp.: | Temperatur Vortemperung [°C] | SDF nach Red. [N] | Durchmesser nach Red. [mm] | SG nach Red. [g/ml] |
|---|---|---|---|---|
| 5.1 | - | 19 | 2,97 | 1,12 |
| 5.2 | 200 | 23 | 2,87 | 1,15 |
| 5.3 | 250 | 22 | 2,89 | 1,17 |

(fortgesetzt)

| Bsp.: | Temperatur Vortemperung [°C] | SDF nach Red. [N] | Durchmesser nach Red. [mm] | SG nach Red. [g/ml] |
|-------|------------------------------|-------------------|----------------------------|---------------------|
| 5.4 | 300 | 17 | 2,88 | 1,21 |
| 5.5 | 350 | 16 | 2,89 | 1,14 |

Aus den Werten ist ein deutlicher Effekt hinsichtlich der Härte (SDF) und des Schüttgewichtes der Katalysatortabletten zu erkennen, die einer Vortemperung bei einer Temperatur im Bereich von 200-300°C unterzogen wurden.

**Beispiel 6:** Bestimmung des Carbonatgehalts der Katalysatortabletten

Die Bestimmung des Carbonatgehalts erfolgt analog der in F. Ehrenberger: "Quantitative Organische Elementaranalyse", VCN Verlagsgesellschaft mbH, Weinheim; Ausgabe 1991; ISBN: 3-527-28056-1, Seite 225 ff beschriebenen Methode.

*Durchführung*

Verwendete Geräte:

IR-Modul, z.B. Fa. Dimatec
Mikrowaage z.B. MT5 / Analysenwaage AT261, Fa. Mettler
Heizplatte
Übliche Laborgeräte

Der in den jeweiligen Probe des Katalysatorformkörpers bzw. der Metallcarbonathaltigen Masse enthaltene anorganische Kohlenstoff, der üblicherweise in Form von Carbonat-, Hydroxycarbonat- und/oder Hydrogencarbonat-Ionen vorliegt, wird unter geringer Wärmezufuhr mit verdünnter Phosphorsäure in Kohlensäure überführt, die zu $CO_2$ und $H_2O$ zerfällt. Das so freigesetzte $CO_2$ wird mit einem Inertgasstrom aus der Probe ausgetrieben. In einer nachgeschalteten Gaswäsche werden störende Komponenten entfernt. Der Gehalt an $CO_2$ in dem Inertgasstrom wird anschließend mittels Infrarotspektroskopischer Messung quantifiziert. Bei dieser Methode liegt die kleinste Menge an anorganischem Kohlenstoff, die sich noch zuverlässig in einer Probe bestimmen lässt, bei etwa 1 mg anorganischem Kohlenstoff pro 100 g Probe.

Die Einwaage der Probe wurde dem erwarteten Gehalt und dem Blindwert des Systems angepasst. Die zur Bestimmung des Carbonatgehalts eingesetzte Menge an Katalysatorprobe lag je nach geforderter Bestimmungsgrenze bei ca. 1 bis 500 mg.

**Beispiel 7:** Hydrierung von $C_{10}$-$C_{18}$-Fettsäuremethylestern

In einem 300 ml Parr-Laborautoklaven mit Katalysatorkorb werden jeweils 10 g des reduzierten und passivierten (red/pass) Katalysators und 200 ml Methylester bei 220°C und 170 bar $H_2$-Druck jeweils 5h gerührt. Die Analyseergebnisse der jeweils direkt nach einer Reaktionszeit von 5h aus dem Reaktionsgemisch entnommenen Proben sind in der nachfolgenden Tabelle 3 dargestellt.

Tabelle 3: Analyseergebnisse der Hydrierung von $C_{10}$-$C_{18}$-Fettsäuremethylestern unter Verwendung der Katalysatoren aus den Beispielen 1, 2 und 3.

| Beispiel | 7.1 *) | 7.2 | 7.3 |
|----------|--------|-----|-----|
| Katalysator | 1 (aus Vergleichsbeispiel 1) | 2 (aus Bsp. 2) | 3 (aus Bsp. 3) |
| Fettalkohole (%) | 71 | 74 | 75 |
| Methylester (%) | 14 | 12 | 12 |
| Wachsester (%) | 14 | 13 | 12 |
| Sonstige (%) | 1 | 1 | 1 |
| *) Vergleichsbeispiel | | | |

16

Man erkennt, dass trotz Einsatz gleicher Mengen an Katalysator die Katalysatoren 2 und 3 mindestens zu der gleichen Aktivität führen wie der Vergleichskatalysator 1 (tendenziell ist die Aktivität sogar erhöht), obwohl diese vor der Tablettierung mit jeweils 15% Kupferpulver "verdünnt" wurden, d.h. mithin bei diesen Beispielen 15% weniger Aktivmasse zur Verfügung steht. Darüber hinaus weisen diese mit Kupferpulver modifizierten Katalysatortabletten nach Reduktion eine deutlich erhöhte Seitendruckfestigkeit auf, was den technischen Einsatz deutlich erleichtert.

Für ein angenommenes Reaktorvolumen von 1 m$^3$ ergeben sich demnach folgende Optionen (Tabelle 4):

Tabelle 4: Einsatzmöglichkeit der Katalysatoren 1, 2 und 3 (Katalysatoren aus den Beispielen 1, 2 und 3) in einem Reaktor mit einem Volumen von 1 m$^3$.

| Katalysator | Katalysatormasse (in Tonnen) | Anteil "inertes" Kupfer (in Tonnen) | Anteil Cu/Zn nach Aktivierung (in Tonnen) |
|---|---|---|---|
| 1 (Grünlinge) | 1,2 | - | 0,84 |
| 1 (red/pass) **) | 0,95 | - | 0,95 |
| 2 (Grünlinge) | 1,72 | 0,26 | 1,02 |
| 2 (red/pass) | 1,25 | 0,26 | 1,02 |
| 3 (red/pass) | 1,3 | 0,23 | 1,07 |
| **) Durchführung von Reduktionsreaktionen nicht möglich, da die Tabletten zu weich sind. | | | |

Man erkennt deutlich die Vorteile der erfindungsgemäß hergestellten Katalysatortabletten (Katalysatoren 2 und 3), da diese für das Befüllen von größeren Mengen Katalysatortabletten in Reaktoren mit großem Volumen (z.B. mit einem Volumen von mehreren m$^3$) geeignet sind und somit Hydrierungen im technischen Maßstab ermöglichen, was mit den Katalysator 1 nicht möglich ist. Des Weiteren lassen sich die Reaktoren mit den erfindungsgemäß hergestellten Katalysatortabletten (Katalysatoren 2 und 3) wesentlich dichter befüllen.

## Patentansprüche

1. Verfahren zur Herstellung eines Katalysatorformkörpers, bei dem man

   a) eine metallcarbonathaltige Masse bereitstellt, die, bezogen auf das Gesamtgewicht der metallcarbonathaltigen Masse,

   - 70 Gew.-% oder mehr eines Metallcarbonatgemisches, enthaltend zwei oder mehr als zwei Metallcarbonate von zwei oder mehr als zwei verschiedenen Metallen (M),
   - 10 bis 25 Gew.-% metallisches Kupfer und
   - 0,5 bis 5 Gew.-% Tablettierhilfsmittel,

   enthält,
   b) aus der in Schritt a) bereitgestellten metallcarbonathaltigen Masse einen Formkörper formt und
   c) den in Schritt b) erhaltenen Formkörper bei einer Temperatur im Bereich von 150 bis 250 °C in Gegenwart von Wasserstoff aktiviert.

2. Verfahren nach Anspruch 1, wobei der in Schritt b) erhaltene Katalysatorformkörper vor der Aktivierung in Schritt c) einen Carbonatgehalt im Bereich von 15 bis 45 Gew.-% aufweist, wobei der Carbonatgehalt wie im Beispiel 6 beschrieben bestimmt wird.

3. Verfahren nach Anspruch 1, wobei der in Schritt b) erhaltenen Formkörper zunächst in einem Schritt c1) einer ersten thermischen Behandlung bei einer Temperatur im Bereich von 150 bis 350 °C in Abwesenheit von Wasserstoff unterworfen wird und der so thermisch behandelte Formkörper anschließend in einem Schritt c2) bei einer Temperatur im Bereich von 150 bis 250 °C in Gegenwart von Wasserstoff aktiviert wird.

4. Verfahren nach Anspruch 3, wobei der in Schritt c1) erhaltene Katalysatorformkörper vor der Aktivierung in Schritt c2) einen Carbonatgehalt im Bereich von 0,1 bis 2,5 Gew.-% aufweist, wobei der Carbonatgehalt wie im Beispiel 6 beschrieben bestimmt wird.

5. Verfahren nach einem der vorangehenden Ansprüche, wobei die Bereitstellung der metallcarbonathaltigen Masse in Schritt a) die folgenden Schritte umfasst

   a1) Bereitstellung eines pulverförmigen Metallcarbonatgemisches, enthaltend zwei oder mehr als zwei Metallcarbonate von zwei oder mehr als zwei verschiedenen Metallen (M), und
   a2) Zugabe des metallischen Kupfers und des Tablettierhilfsmittels zu dem in Schritt a1) bereitgestellten pulverförmigen Metallcarbonatgemisch.

6. Verfahren nach einem der vorangehenden Ansprüche, wobei der Formkörper im Anschluss an die Aktivierung in Schritt c) oder c2) bei einer Temperatur von 60°C oder weniger in Gegenwart eines sauerstoffhaltigen Gasgemisches, insbesondere von Luft, passiviert wird.

7. Verfahren nach einem der Ansprüche 1 bis 6, wobei das in der metallcarbonathaltigen Masse enthaltene Metallcarbonatgemisch, bezogen auf das Gesamtgewicht des Metallcarbonatgemisches,

   - 22 bis 36 Gew.-% Kupfer und
   - 18 bis 33 Gew.-% Zink

   enthält.

8. Verfahren nach einem der Ansprüche 1 bis 7, wobei das Metallcarbonatgemisch Kupfer und Zink enthält, und wobei das Molverhältnis von Kupfer zu Zink im Metallcarbonatgemisch im Bereich von 2 : 1 bis 1 : 1,5 liegt.

9. Verfahren nach einem der vorangehenden Ansprüche, wobei das Metallcarbonatgemisch, ausgenommen von Verunreinigungen, keine weiteren von Kupfer und Zink verschiedene Metalle enthält.

10. Verfahren nach einem der vorangehenden Ansprüche, wobei es sich bei dem metallischen Kupfer um Kupferpulver oder Kupferblättchen handelt.

11. Verfahren nach einem der vorangehenden Ansprüche, wobei das Tablettierhilfsmittel ausgewählt ist unter Graphit, Bornitrid, Molybdändisulfid und Mischungen davon.

**Claims**

1. A process for producing a shaped catalyst body, wherein

   a) a metal carbonate-comprising composition which comprises, based on the total weight of the metal carbonate-comprising composition,

   - 70% by weight or more of a metal carbonate mixture comprising two or more than two metal carbonates of two or more than two different metals (M),
   - 10% to 25% by weight of metallic copper and
   - 0.5% to 5% by weight of tableting aid is provided,

   b) a shaped body is formed from the metal carbonate-comprising composition provided in step a) and
   c) the shaped body obtained in step b) is activated in the presence of hydrogen at a temperature in the range from 150°C to 250°C.

2. The process according to claim 1, wherein the shaped catalyst body obtained in step b) has a carbonate content in the range from 15% to 45% by weight before activation in step c), wherein the carbonate content is determined as described in example 6.

3. The process according to claim 1, wherein the shaped body obtained in step b) is initially subjected to a first thermal treatment at a temperature in the range from 150°C to 350°C in the absence of hydrogen in a step c1) and the shaped body which has been thermally treated in this way is subsequently activated in the presence of hydrogen at a temperature in the range from 150°C to 250°C in a step c2).

4. The process according to claim 3, wherein the shaped catalyst body obtained in step c1) has a carbonate content in the range from 0.1% to 2.5% by weight before activation in step c2), wherein the carbonate content is determined as described in example 6.

5. The process according to any of the preceding claims, wherein the provision of the metal carbonate-comprising composition in step a) comprises the following steps

a1) provision of a pulverulent metal carbonate mixture comprising two or more than two metal carbonates of two or more than two different metals (M) and
a2) addition of the metallic copper and of the tableting aid to the pulverulent metal carbonate mixture provided in step a1).

6. The process according to any of the preceding claims, wherein the shaped body is passivated in the presence of an oxygen-comprising gas mixture, in particular air, at a temperature of 60°C or less after the activation in step c) or c2).

7. The process according to any of claims 1 to 6, wherein the metal carbonate mixture comprised in the metal carbonate-comprising composition comprises, based on the total weight of the metal carbonate mixture,

- 22% to 36% by weight of copper and
- 18% to 33% by weight of zinc.

8. The process according to any of claims 1 to 7, wherein the metal carbonate mixture comprises copper and zinc and wherein the molar ratio of copper to zinc in the metal carbonate mixture is in the range from 2 : 1 to 1 : 1.5.

9. The process according to any of the preceding claims, wherein, with the exception of impurities, the metal carbonate mixture comprises no further metals different from copper and zinc.

10. The process according to any of the preceding claims, wherein the metallic copper is copper powder or copper flakes.

11. The process according to any of the preceding claims, wherein the tableting aid is selected from graphite, boron nitride, molybdenum disulfide and mixtures thereof.


**Revendications**

1. Procédé pour la fabrication d'un corps moulé de catalyseur, dans lequel

a) on prépare une matière contenant des carbonates métalliques qui contient, par rapport au poids total de la matière contenant des carbonates métalliques,

- 70 % en poids ou plus d'un mélange de carbonates métalliques contenant deux ou plus de deux carbonates métalliques de deux ou plus de deux métaux (M) différents,
- 10 à 25 % en poids de cuivre métallique et
- 0,5 à 5 % en poids d'adjuvant de pastillage,

b) on met en forme un corps moulé à partir de la matière contenant des carbonates métalliques préparée dans l'étape a) et
c) on active en présence d'hydrogène le corps moulé obtenu dans l'étape b), à une température dans la plage de 150 à 250 °C.

2. Procédé selon la revendication 1, dans lequel le corps moulé de catalyseur obtenu dans l'étape b) présente, avant l'activation dans l'étape c), une teneur en carbonates dans la plage de 15 à 45 % en poids, la teneur en carbonates étant déterminée comme décrit dans l'exemple 6.

3. Procédé selon la revendication 1, dans lequel le corps moulé obtenu dans l'étape b) est d'abord soumis, dans une étape c1), à un premier traitement thermique à une température dans la plage de 150 à 350 °C en l'absence d'hydrogène, et le corps moulé ainsi traité thermiquement est ensuite activé en présence d'hydrogène, dans une étape c2), à une température dans la plage de 150 à 250 °C.

**4.** Procédé selon la revendication 3, dans lequel le corps moulé de catalyseur obtenu dans l'étape c1) présente, avant l'activation dans l'étape c2), une teneur en carbonates dans la plage de 0,1 à 2,5 % en poids, la teneur en carbonates étant déterminée comme décrit dans l'exemple 6.

**5.** Procédé selon l'une quelconque des revendications précédentes, dans lequel la préparation de la matière contenant des carbonates métalliques comprend dans l'étape a)les étapes suivantes

a1) préparation d'un mélange pulvérulent de carbonates métalliques, contenant deux ou plus de deux carbonates métalliques de deux ou plus de deux métaux (M) différents, et
a2) addition du cuivre métallique et de l'adjuvant de pastillage au mélange pulvérulent de carbonates métalliques préparé dans l'étape a1).

**6.** Procédé selon l'une quelconque des revendications précédentes, dans lequel le corps moulé, à la suite de l'activation dans l'étape c) ou c2), est passivé à une température de 60 °C ou moins en présence d'un mélange gazeux contenant de l'oxygène, en particulier d'air.

**7.** Procédé selon l'une quelconque des revendications 1 à 6, dans lequel le mélange de carbonates métalliques contenu dans la matière contenant des carbonates métalliques contient, par rapport au poids total du mélange de carbonates métalliques,

- 22 à 36 % en poids de cuivre et
- 18 à 33 % en poids de zinc.

**8.** Procédé selon l'une quelconque des revendications 1 à 7, dans lequel le mélange de carbonates métalliques contient du cuivre et du zinc, et dans lequel le rapport molaire de cuivre à zinc dans le mélange de carbonates métalliques se situe dans la plage de 2 : 1 à 1 : 1,5.

**9.** Procédé selon l'une quelconque des revendications précédentes, dans lequel le mélange de carbonates métalliques, à l'exception d'impuretés, ne contient pas d'autres métaux différents du cuivre et du zinc.

**10.** Procédé selon l'une quelconque des revendications précédentes, dans lequel pour ce qui est du cuivre métallique il s'agit de poudre de cuivre ou de paillettes de cuivre.

**11.** Procédé selon l'une quelconque des revendications précédentes, dans lequel l'adjuvant de pastillage est choisi parmi le graphite, le nitrure de bore, le bisulfure de molybdène et des mélanges de ceux-ci.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102014004413 A1
- EP 2096098 A1
- DE 102012019123 A1

- DE 10357715 A1
- WO 0117934 A1
- DE 102014004413